# DEMANDE DE BREVET EUROPEEN

(11) **EP 2 423 318 A1**
(43) Date de publication de la demande: **29.02.2012**
(21) Numéro de dépôt: 11172720.2
(22) Date de dépôt: 15.12.2008
(51) Int. Cl.: C12P 7/46, C07C 51/43, C07C 51/41, C07C 51/02

(54) **Procedes de production d'acide succinique**

(30) Priorité: 13.12.2007 FR 0759827; 18.02.2008 FR 0851028
(62) Demande divisionnaire de: 08865165.8
(71) Demandeur: Roquette Frères, 62136 Lestrem (FR)
(72) Inventeur: Dehay, Frédéric, 62840 LAVENTIE (FR); Segueilha, Laurent, 59350 SAINT ANDRE LEZ LILLE (FR); Calande, Olivier, 59280 ARMENTIERES (FR); Varlamoff, Caroline, 59112 ANNOEULIN (FR)
(74) Mandataire: Cabinet Plasseraud

(57) **Abrégé**

L'invention concerne un procédé de production d'acide succinique, comprenant :
(a) une étape de culture d'une souche *d'Escherichia coli* en conditions aérobies, lors de laquelle le pH est régulé par ajout, dans le milieu de culture, d'un composé de magnésium,
(b) une étape de production d'ions succinate par fermentation de la souche cultivée à l'étape (a) en conditions anaérobies en présence de CO₂,
(c) une étape de conversion des ions succinate formés à l'étape (b) en acide succinique.

## Description

La présente invention concerne des procédés de production d'acide succinique et/ou d'ions succinate par fermentation en conditions anaérobies.

L'acide succinique (ou acide butanedioïque) est un acide organique avec deux groupes carboxyles, de formule semi-développée COOH-CH₂-CH₂-COOH, qui intervient dans le métabolisme cellulaire, comme intermédiaire métabolique du cycle de Krebs dans la mitochondrie.

Il trouve de nombreuses applications dans les domaines cosmétiques, agroalimentaires, pharmaceutiques, textiles et dans les plastiques. Ainsi il est par exemple utilisé comme intermédiaire de synthèse des plastiques pour la fabrication de 1,4 butanediol, de tétrahydrofuranne et de gammabutyrolactone.

De nouveaux produits dérivés de l'acide succinique sont en constant développement, incluant le développement de polyesters.

Généralement, les esters de l'acide succinique ont le potentiel d'être de nouveaux solvants "verts" qui peuvent substituer les solvants plus nuisibles pour l'homme et l'environnement.

La production d'acides carboxyliques, tels que l'acide malique, l'acide succinique ou l'acide fumarique. à partir de matières premières renouvelables (en l'occurrence par le biais des processus de fermentation) est connue de l'Homme du métier.

Le succinate est un intermédiaire métabolique lors de la fermentation anaérobie par des bactéries produisant du propionate, mais ces procédés de fermentation résultent en la production de très faibles rendements et titres en acide succinique.

Ces dernières années, beaucoup de microorganismes producteurs d'acide succinique ont été isolés, comme par exemple les bactéries anaérobies du rumen, *Bacteroides ruminicola* et *Bacteroides amylophilus.* Toutefois, les organismes du Rumen sont très instables lors des procédés de fermentation, est ne sont donc pas utilisables de manière industrielle pour la production d'acide succinique.

On saint depuis longtemps qu'un mélange de plusieurs acides, dont l'acide succinique est produit à partir de la fermentation de *E*. *coli* en présence de glucose et de CO₂ comme substrats carbonés, comme décrit par JL Stokes en 1949 «Fermentation of glucose by suspensions of Escherichia coli » J. Bacteriol., 57: 147-158. Cependant, pour chaque mole de glucose fermentée, seule 0.3 à 0.4 Moles d'acide succinique sont produites. Des études ont donc été menées sur des bactéries, en particulier *Escherichia coli,* modifiées génétiquement de manière à inactiver les voies métaboliques consommant le NADH nécessaire à la fabrication de l'acide succinique et de manière à activer les voies métaboliques de production du succinate (sel de l'acide succinique).

En effet, le chemin métabolique fermentaire qui permet la conversion de l'oxaloacétate en malate, puis famarate et enfin succinate nécessite deux moles de NADH par mole de succinate produit. Le verrou métabolique majeur pour la production de succinate et donc la biodisponibilité cellulaire en NADH.

Pour solutionner cette difficulté, le document US 7.223.567 décrit l'utilisation d'une souche *Escherichia coli* recombinante surproductrice de succinate pour la même quantité de NADH disponible.

Cette souche *Escherichia coli* SBS550 MG pHL 413 présente une inactivation des produits des gènes *adhE*, *ldhA* (impliqués dans les voles consommatrices de NADH), et une inactivation des produits des gènes *ack*-*pta* et du gène *iclR* (activant la voie glyoxylate), et contient un vecteur plasmidique surexprimant un gène PYC exogène. L'article de Sanchez et al. (intitulé «Novel pathway engineering design of the anaerobic central metabolic pathway in Escherichia coli to increase succinate yield and productivity » in Metabolic Engineering 7 (2005) 229-239), le brevet américain US 7 223 567 et la demande de brevet américain US 2005/0042736 ont développé de nouvelles conditions de culture et de production associées à cette souche pour améliorer ses rendements de production en acide succinique.

L'homme du métier recherche constamment de nouveaux procédés améliorés de production d'acide succinique. En particulier, l'homme du métier cherche à optimiser le rendement et la productivité obtenus. Par ailleurs, les méthodes de fermentation classiques conduisent à d'importants rejets en dioxyde de carbone dans l'atmosphère, ce qui est bien évidemment peu désirable.

Selon un aspect, la présente invention concerne un procédé de production d'acide succinique et/ou d'ions succinate par fermentation anaérobie d'une souche d'*Escherichia* c*oli,* comprenant :
(A) une étape de fermentation d'une source de carbone dans un fermenteur, avec apport de CO₂, réalisée avec évents du fermenteur fermés de manière à ce que rapport de CO₂ soit asservi à la consommation de CO₂ par la souche : suivie, avant épuisement total de la source de carbone, d'
(B) une étape de fermentation de la source de carbone restante, sans apport de CO₂, de manière à consommer le CO₂ résiduel.

L'homme du métier est familier avec les techniques de fermentation (telles que notamment décrites dans Fermentation & Biochemical Engineering Handbook : principles, process design & equipment, 2nd ed 1996 de VOGEL Henry C. et TODARO Celeste L.).

La fermentation est une réaction biochimique qui consiste généralement à Libérer de l'énergie, ou à produire certains métabolites d'intérêt, à partir d'un substrat organique sous l'action d'enzymes microbiennes.

La fermentation est généralement conduite dans des dispositifs (fermenteurs) adaptés au processus de fermentation, c'est-à-dire adaptés à la culture de microorganismes dans les conditions voulues (dispositifs permettant le cas échéant de contrôler les équilibres gazeux du milieu de culture, notamment par des conduits d'entrée et/ou de sortie en gaz, évents, etc, dispositifs permettant l'introduction de milieu de culture et autres substances ; dispositifs permettant de contrôler, réguler, modifier d'autres types de paramètres, tels que l'agitation, la température, le pH, etc).

L'homme du métier est également familier avec la fermentation en conditions anaérobies. Selon la présente invention, ceci désigne des conditions de culture en l'absence d'oxygène. De manière préférée, des conditions de culture anaérobies sont des conditions de culture en présence de dioxyde de carbone. Selon un mode de réalisation, les conditions de fermentation anaérobies en présence de CO₂ et/ou avec apport de CO₂ sont des conditions de fermentation en saturation de CO₂.

Par « avant épuisement total de la source de carbone » lors de l'étape (A), on entend un instant de l'étape (A) où le milieu de fermentation contient une quantité résiduelle en source de carbone pouvant être intégralement transformée par la souche en acide succinique et/ou succinate grâce au CO₂ disponible en solution à cet instant (sous forme de CO₂ dissous ou de HCO₃).

Lors de l'étape (A), le(s) évent(s) du fermenteur étant fermé(s) et l'alimentation en CO₂ du fermenteur étant maintenue, l'apport de CO₂ est réalisé de manière discontinue, automatiquement ajusté en fonction de la consommation du CO₂ par la souche pour la production d'acide succinique et/ou succinate.

Par « automatiquement ajustée », on entend, compte tenu de l'équilibre thermodynamique entre la phase liquide (milieu de fermentation) et la phase gazeuse (« atmosphère ») présentes dans le fermenteur (évent(s) fermé(s)), qu'un apport d'une quantité donnée de CO₂ ne peut intervenir que suite à la consommation d'une quantité équivalente par la souche par fermentation (et donc la production concomitants d'acide succinique et/ou succinate).

Lors de l'étape (B), il n'y a pas d'apport de CO₂, ce qui signifie que l'apport en CO₂ réalisée lors de l'étape (A) est interrompu. Ceci peut notamment être réalisé par coupure de l'alimentation en CO₂.

Avantageusement selon l'invention, la fermentation lors de l'étape (B) consomme le CO₂ (résiduel dissous) et les ions HCO₃⁻ présents dans le milieu de fermentation.

Selon un mode préféré de réalisation, avant le début de l'étape (A), l'apport de CO₂ est réalisé par injection, avec évents du fermenteur ouverts, de manière à atteindre la saturation du milieu de fermentation en CO₂.

A titre d'exemple, le CO₂ peut être introduit par injection à un débit de 0,15-0,40 vvm (volume de CO₂ par volume de culture par minute), de préférence 0,3 vvm.

Par « milieu de fermentation saturé en CO₂ », on entend le fait que le milieu de culture contient la quantité maximale en CO₂ qui peut y être dissous dans les conditions correspondantes (température, pH, etc). Par exemple ceci peut correspondre à une concentration de 1-2 g/L. par exemple de l'ordre de 1,5 g/L à 37°C, pH 7.

Selon un mode de réalisation, l'étape (A) et/ou l'étape (B) se déroule à un pH dans un intervalle de 6,0-7,0, de préférence 6,4-6,8, de préférence 6,5-6,6.

Selon un mode de réalisation, la source de carbone est le glucose.

Selon un mode de réalisation, au début de l'étape (A), le milieu de fermentation comprend 15-40 g/L, de préférence 15-25 g/L, de préférence 15-20 g/L de glucose. Selon un mode de réalisation, au début de l'étape (B), le milieu de fermentation comprend 2-6 g/L, de préférence environ 4 g/L de glucose.

Selon un mode de réalisation préféré, la souche d'*Escherichia Coli* est une souche qui possède le génotype Δ*adhE* Δ*ldhA* Δ*ielR* Δ*ackpta* PYC. Ce génotype permet avantageusement de favoriser la production d'acide succinique par fermentation en présence de CO₂. Le symbole Δ indique que le gène en question a été inactivé, par exemple par mutation, délétion, interruption, insertion, ou « down »-régulation, par exemple par introduction d'un codon stop, insertion ou délétion résultant en un changement du cadre de lecture, mutation ponctuelle, etc.

Le génotype Δ*adhE* Δ*ldhA* Δ*iclR* Δ*ackpta* PYC correspond donc à :
- Δ*adhE* : inactivation de l'alcool déshydrogénase ;
- Δ*ldhA* : inactivation de la lactate déshydrogénase;
- Δ*iclR* : inactivation de l'isocitrate lyase (également connue sous le nom de aceA)
- Δ*ackpta* :inactivation de l'acétate kinase-phosphotransacétylase ;
- PYC ; expression d'un gène de pyruvate carboxylase. Ceci indique que la souche exprime le gène PYC, par exemple grâce à une transformation par un plasmide portant un exemplaire fonctionnel de ce gène, ou par intégration génomique d'un exemplaire fonctionnel de PYC. Le gène PYC est avantageusement le gène pyc de *Lactococcus lactis.*

Selon un mode de réalisation très préféré, la souche d'*Escherichia Coli* est la souche SBS550MG - pHL413. Cette souche est décrite dans Sanchez et al., Metabolic Engineering, 7 (2005) 229-239, et dans les documents US 7,223,567 et US 2005/0042736.

Selon un aspect, la présente invention concerne un procédé de production d'acide succinique, comprenant:
(a) une étape de culture d'une souche d'*Escherichia coli* en conditions aérobies, lors de laquelle le pH est régulé par ajout, dans le milieu de culture, d'un composé de magnésium,
(b)une étape de production d'ions succinate par fermentation de la souche cultivée à l'étape (a) en conditions anaérobies en présence de CO₂,
(c) une étape de conversion des ions succinate formés à l'étape (b) en acide succinique.

L'homme du métier est également familier avec la fermentation et la culture en conditions aérobies. Selon la présente invention, ceci désigne des conditions de culture en présence d'oxygène. Selon un mode de réalisation, l'oxygène provient de l'atmosphère.

A l'étape (a)., il y a ainsi croissance et propagation de la souche d'*E*. *coli*. Il y a ainsi production de biomasse, c'est-à-dire accroissement de la population de cellules. Cette étape peut typiquement comporter des sous étapes de pré-culture.

Par « réguler le pH », on entend selon la présente invention, l'action de maintenir la valeur du pH du milieu de culture dans un certain intervalle ou sélection de valeur. Selon l'invention, le pH peut être régulé de différentes façons :
- régulation dans un intervalle : la valeur du pH est maintenue dans un certain intervalle de valeurs. La valeur du pH peut alors varier au cours du temps, sans toutefois sortir de l'intervalle considéré ;
- régulation «point bas» : la valeur du pH est maintenue au dessus d'une valeur seuil. La valeur du pH peut alors varier au cours du temps, sans toutefois descendre en deçà de la valeur seuil ;
- régulation à une valeur unique: la valeur du pH est maintenue à cette valeur de manière constante au cours du temps.

Par « ajout d'un composé », on entend l'introduction du composé dans le milieu de culture. L'ajout peut se faire selon différentes modalités : ajout d'une suspension et/ou ajout d'une solution et/ou ajout d'un solide (par exemple sous forme de pondre).

Selon un mode de réalisation, le composé de magnésium de l'étape (a), est choisi parmi l'oxyde de magnésium, l'hydroxyde de magnésium, et le carbonate de magnésium. L'oxyde de magnésium, l'hydroxyde de magnésium ou le carbonate de magnésium peuvent être ajoutés sous forme de poudre ou sous terme de suspension, typiquement de suspension aqueuse, par exemple à des concentrations de 20 % à 30 % p/v.

Selon un mode de réalisation, les étapes (a) et/ou (b) sont réalisées dans un milieu contenant la source de carbone utilisée, typiquement du glucose, et notamment à des concentrations de 10-30 g/L, par exemple 20 g/L.

Selon mode de réalisation, lors de l'étape (b), le pH est régulé par ajout, dans le milieu de fermentation, d'un composé choisi dans le groupe constitué des composés du magnésium (par exemple choisi parmi l'oxyde de magnésium, l'hydroxyde de magnésium, et le carbonate de magnésium), des composés du calcium (par exemple choisi parmi l'oxyde de calcium, l'hydroxyde de calcium, et le carbonate de calcium, des composés du potassium (par exemple choisi parmi l'hydroxyde de potassium, et le carbonate de potassium), des composés d'ammonium (par exemple choisi parmi l'hydroxyde d'ammonium, et le carbonate d'ammonium), et des composés du sodium (par exemple choisi parmi l'hydroxyde de sodium, et le carbonate de sodium,), et de leurs mélanges.

Selon un mode de réalisation, l'étape (b) se déroule à un pH dans un intervalle de 6,0-7,0, de préférence 6,4-6,8.

Selon un mode de réalisation, l'étape (c) comprend une acidification. L'acidification peut notamment être réalisée par ajout d'au moins un acide choisi parmi l'acide ortho-phosphorique, l'acide oxalique, l'acide sulfurique.

Selon un mode de réalisation, lors de l'étape (b), le pH est régulé par ajout, dans le milieu de fermentation, d'un composé choisi dans le groupe constitué des composés du magnésium, formant ainsi du succinate de magnésium.

Dans ce cas, l'étape (c) peut comprendre :
(c-1) une étape de conversion de succinate de magnésium formé à l'étape (b) en succinate de sodium, et
(c-2) une étape de conversion, par électrodialyse bipolaire, du succinate de sodium formé à l'étape (c-1) en acide succinique.

L'étape (c-1) peut typiquement être réalisée par ajout de carbonate de sodium. Le carbonate peut être ajouté sous forme de solution ou de poudre, typiquement de solution aqueuse, par exemple à des concentrations de 1 à 2 M. Le carbonate de magnésium, insoluble, précipite. Le carbonate de magnésium peut être traité dans un tour à haute température, par exemple un four à > 700°C. Il en résulte du MgO et du CO₂, dont l'un au moins peut être recyclé.

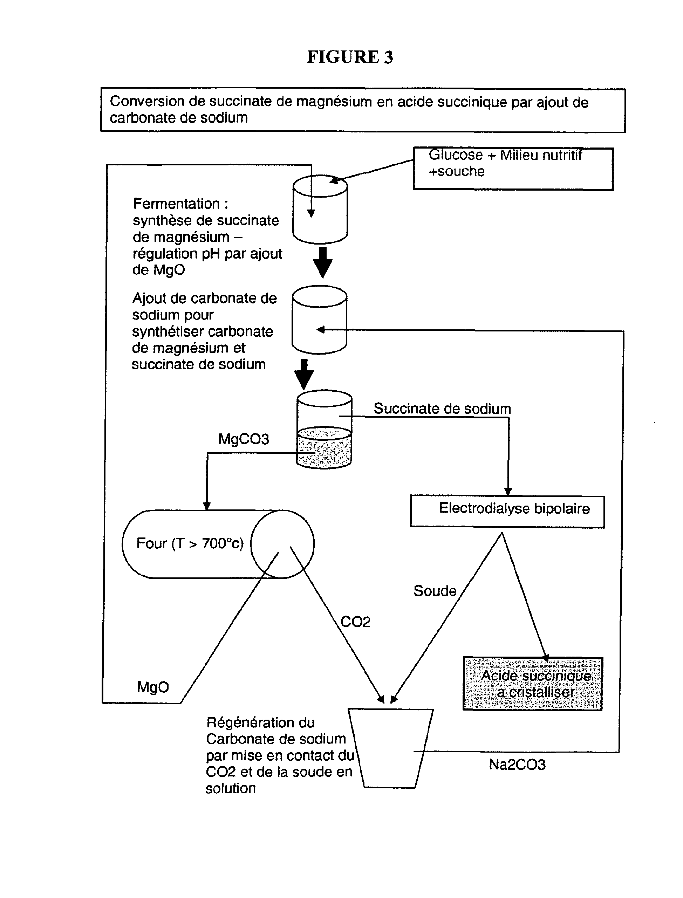

Le carbonate de magnésium peut être alternativement valorisé en tant que tel. Le succinate de sodium peut avantageusement être traité par électrodialyse bipolaire (ce qui n'est pas le cas du succinate de magnésium), donnant de la soude et de l'acide succinique, qui peut être cristallisé. La technique d'électrodialyse bipolaire est par ailleurs bien connue de l'homme du métier. La soude produite peut le cas échéant être reconvertie, avec le CO₂ précédemment émis du four haute température, pour former du carbonate de sodium. L'ensemble des étapes est représenté à la Figure 3.

Selon un mode de réalisation préféré, la souche d'*Escherichia Coli* est une souche qui possède le génotype Δ*adhE* Δ*ldhA* Δ*iclR* Δ*ackpta* PYC. Selon un mode de réalisation très préféré, la souche d'*Escherichia Coli* est la souche SBS550MG - pHL413.

Selon un autre aspect, la présente invention concerne un procédé de production d'acide succinique, comprenant :
(i) une étape de production de succinate de magnésium par fermentation en conditions anaérobies d'une souche d'*Escherichia Co*/*i* en présence de CO₂, lors de laquelle le pH est régulé par ajout, dans le milieu de fermentation, d'un composé de magnésium, et
(ii) une étape de conversion du succinate de magnésium formé à l'étape (i) en acide succinique.

Les expressions « réguler le pH », « ajout d'un composé », sont définies ci-dessus. Selon un mode de réalisation, le composé de magnésium de l'étape (i) est choisi parmi l'oxyde de magnésium, l'hydroxyde de magnésium, et le carbonate de magnésium. De préférence, il s'agit de l'oxyde de magnésium (magnésie, de formule MgO).

L'oxyde de magnésium, l'hydroxyde de magnésium ou le carbonate de magnésium peuvent être ajoutés sous forme de poudre ou sous forme de suspension, typiquement de suspension aqueuse, par exemple à des concentrations de 20 % à 30 % p/v.

Selon un mode de réalisation, l'étape (i) se déroule à un pH dans l'intervalle 6,0-7,0, de préférence 6,4-6,8, de préférence 6,5-6,6.

Selon un mode de réalisation, les étapes (i) et (ii) sont réalisées dans un milieu contenant la source de carbone utilisée, typiquement du glucose, et notamment à des concentrations de 10~30 g/L, par exemple 20 g/L.

Selon un mode de réalisation, l'étape (ii) comprend une acidification. Cette acidification peut être réalisée de différentes manières. Selon un mode de réalisation, l'acidification est réalisée par ajout d'au moins un acide choisi parmi l'acide ortho-phosphorique, l'acide oxalique, et l'acide sulfurique. Ces acides peuvent être ajoutés purs ou sous forme de solutions aqueuses concentrées.

Selon un autre mode de réalisation, l'étape (ii) comprend ;
(ii-a) une étape de conversion du succinate de magnésium formé à l'étape (i) en succinate de sodium, et
(ii-b) une étape de conversion, par électrodialyse bipolaire, du succinate de sodium formé à l'étape (ii-a) en acide succinique.

Ces étapes ont été décrites ci-dessus pour les étapes (c-1) et (c-2).

Selon un mode de réalisation préféré, la souche d'*Escherichia coli* est une souche qui possède le génotype Δ*adhE* Δ*dhA* Δ*iclR* Δ*ackpta* PYC. Selon un mode de réalisation très préféré, la souche d'*Escherichia Coli* est la souche SBS550MG - pHL413. Selon un autre aspect, la présente intention concerne un procédé d'obtention d'acide succinique comprenant :
- un procédé de production d'acide succinique et/ou d'ions succinate, par exemple choisi parmi ceux décrits ci-dessus ;
- une étape d'acidification des ions succinate en acide succinique par exemple par ajout d'acide fort dans le moût,
- optionnellement une étape de purification de l'acide succinique ; et
- une étape de cristallisation de l'acide succinique.

Selon un mode de réalisation, dans tous les procédés décrits ci-dessus, l'étape de purification comprend une purification éthanolique qui se déroule comme suit :
- filtration (élimination d'un précipité protéique), par exemple sur Büchner et/ou sur terre de filtration du mout acidifié,
- optionnellement, concentration du filtrat par évaporation sous vide (de préférence, suivant un facteur de concentration entre environ 2 et 8).
- ajout d'éthanol, par exemple de l'éthanol 95%, dans un rapport 1/1, à 5/1, pour provoquer la précipitation des sels (l'acide succinique reste soluble),
- séparation du précipité salin par filtration, par exemple sur une membrane,
- récupération de l'éthanol par évaporation sous vide,
- traitement sur charbon actif, puis filtration sur plaque et terre de filtration,

La Figure 1 illustre les performances en production d'acide succinique par fermentation anaérobie en fonction du composé utilisé pour réguler le pH.

La Figure 2 compare les cinétiques de production selon que MgO ou NaOH est utilisé pour réguler le pH.

La Figure 3 représente un mode de réalisation pour la production d'acide succinique : conversion de succinate de magnésium en acide succinique par ajout de carbonate de sodium.

L'invention est illustrée par les exemples de réalisation ci-dessous, qui sont non-limitatifs.

### Exemples

### Exemple 1 : Production d'acide succinique par fermentation anaérobie selon deux modes différents d'apport du CO₂ avec évent du fermenteur ouvert ou fermé

Le procédé de production d'acide succinique comprend :
- une phase de préculture en Erlenmeyer,
- une phase de culture en conditions aérobies dans un milieu de culture comprenant du corn steep comme source d'azote et du glucose comme source de carbone, cette phase permettant ta production de biomasse, et
- une phase anaérobie permettant la production proprement dite en acide succinique. Les phases en conditions aérobie et anaérobie sont réalisées dans un même fermenteur. La souche utilisée est la souche SBS550MG-pHL413.

### Phase aérobie :

La souche SBS550MG-pHL413 est précultivée en Erlenmeyer pendant 17h à 37°C, sous agitation à 125 rpm. 400 ml de milieu sont inoculés par la souche dans un Erlenmeyer de 2 litres à 2 chicanes.

La composition de ce milieu de préculture est la suivante :

| | |
|---|---|
| tryptone | 10g/L |
| extrait de levure | 5g/L |
| NaCl | 10 g/L |
| Antibiotiques (ampicilline, carbenicilline, oxacilline) | 67 mg/L |

La souche ainsi précultivée est placée dans un fermenteur de 15L dans un milieu de culture dont la composition est la suivante :

| **Glucose**: 2 g/L départ + 2 g/L lorsque les premiers 2g/L sont consommés | |
|---|---|
| **Corn steep** : 60 g/L | |
| **Sels et antibiotiques**: | g/L |
| (NH₄)₂SO₄ | 0,25 |
| K₂HPO₄ | 0.7 |
| KH₂PO₄ | 1.2 |
| KCl | 2 |
| CaCl₂ | 0,2 |
| MgSO₄ | 0,25 |
| Ampicilline | 0,067 |
| Biotine | 0.001 |
| Thiamine | 0.001 |

L'inoculum obtenu par préculture en Erlenmeyer représente 3% du volume total du milieu cultivé dans le fermenteur.

Les conditions de culture lors de la phase aérobie sont une température de 37°C, une agitation de 500 rpm, une aération de 1 vvm et pas de régulation de pH (le pH est simplement ajusté à 7,5 avant stérilisation du milieu).

### Phase anaérobie :

### ○ Protocole avec apport continu de CO₂

- On ajoute dans le milieu glucose : 20 g/L départ + 15 g/L à 24h + 4 g/L à 50h.
- La fermentation est réalisée à pH 6,4 avec une injection continue de CO₂ à un débit de 03 vvm (L/L/min), à 37°C, évent du fermenteur ouvert, sous agitation 250 rpm.
- Elle se termine en 63,5 h avec un titre final en acide succinique de 30 g/L dans le milieu de culture.
- La quantité globale de CO₂ consommée s'établit à (0,3 vvm x 60 min x 63,3 h / 22,4 mol/L x 44 g/mol) 2245 g/L, soit 73 g/g d'acide succinique formé.
- Par ailleurs, une concentration de 2 g/L d'HCO₃ (correspondant à 1,5 g/L de CO₂) est présente en fin de fermentation dans le milieu de culture.

### ○ Protocole selon l'invention

Le protocole de fermentation est identique à celui ci-dessus, excepté que
- au début de la phase anaérobie, du CO₂ est introduit dans le fermenteur à un débit de 0.3 vvm pendant 1 minute de façon à chasser l'air résiduel issu de la phase aérobie,
- la pression du réseau de CO₂ est réduite à 0,4 bar,
- l'event du fermenteur est fermé hermétiquement de façon à empêcher la sortie du CO₂,
- ainsi, l'injection de CO₂ est précisément ajustée à sa consommation tout au long de la fermentation,
- l'injection de CO₂ est coupée lorsque la concentration résiduelle en glucose atteint 4 g/L de façon à consommer l'HCO₃⁻ résiduel dissous dans le milieu.

### Résultats selon l'invention

- concentration finale en acide succinique produit dans le milieu de culture en fin de fermentation : 30 g/L, ce qui est identique à la concentration obtenue avec apport continu en CO₂.
- concentration de HCO₃⁻ résiduel dans le milieu de culture en fin de fermentation : 0,3 g/L (ce qui représente une réduction de 85% par rapport à la concentration obtenue avec apport continu en CO₂).
- consommation de CO₂ : 0,59 g/l au départ + 6 g/l fixé sur le succinique, soit 0,2 g / g d'acide (ce qui représente une réduction de 99,7% par rapport à la concentration obtenue avec apport continu en CO₂)

Ainsi, avantageusement selon l'invention, tout en maintenant le rendement de production d'acide succinique, on évite d'importants rejets en dioxyde de carbone:
- le fait de travailler en réacteur clos limite naturellement les rejets, et
- par ailleurs, on observe une diminution de la concentration en HCO₃⁻ résiduel dans le milieu de culture en fin de fermentation. Or, l'acidification de l'HCO₃⁻ résiduel dans le milieu de culture en fin de fermentation conduit à un dégagement de CO₂.

### Exemple 2 : Production d'acide succinique par fermentation anaérobie avec un milieu minéral et régulation du pH avec différents composés, dont un à base de magnésium

La souche utilisée est la souche SBS550MG - pHL413.

Lors de la phase de fermentation en conditions anaérobies, et lors de la phase de fermentation en conditions anaérobies, le pH est régulé à une valeur de 6,75 en utilisant différents composés: NaOH, NH₃, KOH, CaO. ou MgO.

Le schéma du protocole est le suivant :
- Pré culture en Erlenmeyer;
- Subculture en fermenteur ;
- Production en fermenteur : 2 phases :
   o Phase aérobie : production de biomasse,
   o Phase anaérobie production d'acide succinique en présence de CO₂.

Chaque étape est détaillée ci-dessous :

### Préculture en Erlenmeyer

| **Milieu** | g/L |
|---|---|
| Tryptone | 10 |
| Extrait de levure | 5 |
| NaCl KH₂PO₄ | 10 3 |
| Na₂HPO₄ | 6 |
| NH₄Cl | 1 |
| MgSO₄, 7H₂O | 0,25 |
| Nacl | 0,5 |
| | |
| antibiotiques | 67 mg/L |
| (ampicilline, carbenicilline, oxacilline) | |

- Incubation à 37°C pendant < 24 h ;
- agitation : 125 rpm :
- volume : 500 ml dans Erlenmeyer 2L.

### subculture en fermenteur

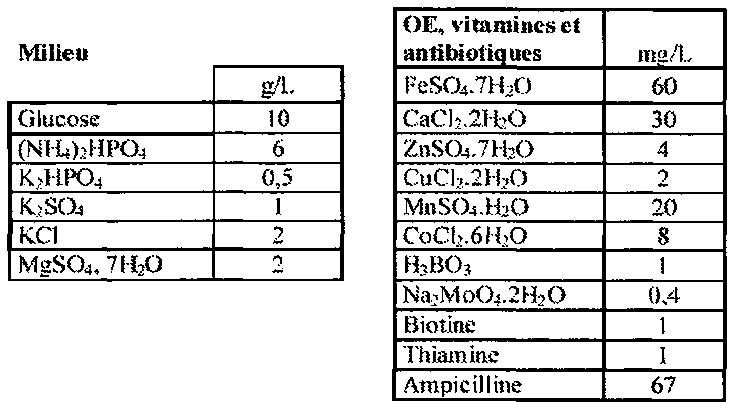

- Inoculum 6 % :
- 37°C, agitation; 450 rpm, aération : 1 vvm ;
- pH régulé à 6,75 par NaOH 5N ;
- Durée : > 20 h.

### Production en fermenteur : 2 phases

### Phase aérobie : production de biomasse

### Milieu

**Glucose :** 10 g/L départ + 10 g/L quand les premiers 10 g/L sont consommés

| **Sels:** | g/L |
|---|---|
| (NH₄)₂HPO₄ | 6 |
| K₂HPO₄ | 0,5 |
| K₂SO₄ | 1 |
| KCl | 2 |
| MgSO₄, 7H₂O | 2 |

- Oligo Eléments (OE) et vitamines : idem subculture
- Inoculum 13% ;
- 37°C, agitation : 450 rpm, aération ; 1 vvm ;
- Régulation pH à 6,75 par ajout de NaOH 5N, respectivement NH₃ 28 % p/v, respectivement KOH 5N, respectivement CaO 20 % p/v, respectivement MgO 20 % p/v.

### Phase anaérobie ; production succinique avec apport de CO₂

- ajout de glucose 20 g/L ;
- injection CO₂ à 0,2 vvm ;
- 37°C, agitation 250 rpm ;
- Régulation pH à 6,75 par ajout de NaOH 5N, respectivement NH₃ 28 % p/v, respectivement KOH 5N, respectivement CaO 20 % p/v, respectivement MgO 20 % p/v.

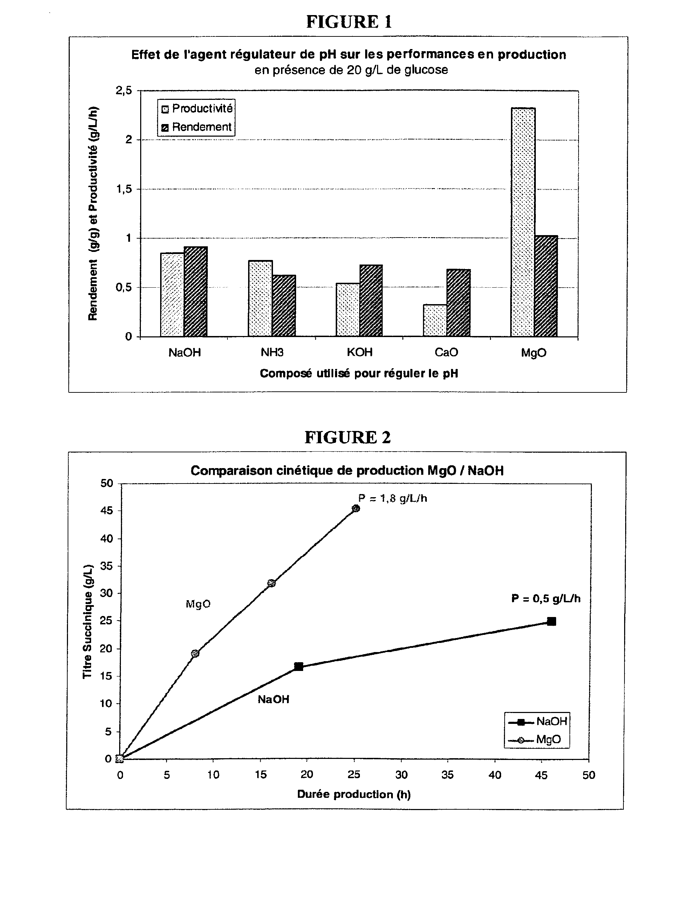

Pour chaque protocole, la quantité d'acide succinique produit est mesurée par HPLC. Les résultats sont représentés en Figure 1, avec les productivités et les rendements obtenus sur des phases de production courtes correspondant à la consommation de 20 g/L de glucose.

De façon surprenante et avantageuse selon l'invention, pour la régulation du pH lors de la fermentation anaérobie, l'utilisation de MgO donne de loin les meilleures performances avec un rendement supérieur à 100% et une productivité volumique 2,5 fois supérieure à celle obtenue avec la plus efficace des autres bases (NaOH).

Le tableau ci-dessous complète la comparaison en montrant que l'utilisation de MgO donne en outre le meilleur rendement de production de biomasse, et la plus faible synthèse de co-produit.

| | **Rendement Biomasse** | **Productivité volumique Acide Succinique** | **Rendement Acide Succinique** | **Productivité spécifique Acide Succinique** | **Co-produits** |
|---|---|---|---|---|---|
| | Y x/g | PV sa | Y sa/g | PS sa | Acides malique + pyruvique |
| | % glucose aérobie | g/L/h anaérobie | % glucose anaérobie | g/g/h anaérobies | % succinique |
| **NAOH** | 25 | 0.9 | 91 | 0.24 | 26 |
| **NH3** | 29 | 0.8 | 62 | 0.20 | 22 |
| **KOH** | 25 | 0,5 | 72 | 0.16 | 48 |
| **CaO** | 32 | 0,3 | 68 | 0,12 | 50 |
| **MgO** | 35 | 2,3 | 103 | 0.48 | 21 |

| | | | | | |
|---|---|---|---|---|---|
| sa = acide succinique, | | | | | |

### Cinétique de production : comparaison de la régulation du pH par ajout de MgO et par ajout de NaOH

La Figure 2 compare l'évolution du titre en acide succinique obtenu en prolongeant les phases de production en présence de soude ou de magnésie.

Cette comparaison révèle un autre avantage inattendu de l'utilisation de MgO : le faible ralentissement de la cinétique au cours de l'accumulation de l'acide succinique. Avantageusement l'utilisation de MgO permet d'accroître la productivité et la vitesse de production d'acide succinique par rapport à l'utilisation de NaOH. En outre, l'utilisation de MgO permet d'atteindre des concentrations (titres) en acide succinique supérieures à 50 g/L.

### Exemple 3: Production d'acide succinique et régulation du pH en phase de croissance aérobie à l'aide d'un composé de magnésium

La souche utilisée est la souche SBS550MG ... pHL413.

Le protocole mis en oeuvre pour la préculture et la subculture est identique à celui décrit dans l'exemple 2.

Pour la production en fermenteur, selon l'invention, la régulation du pH pendant la phase de culture aérobie (croissance de la souche, production de biomasse) est réalisée à l'aide de MgO, tandis que la régulation de pH pendant la phase de production de succinate en conditions anaérobies est réalisée à l'aide de soude.

### Les autres conditions de mise en oeuvre sont identiques à cel les de l'exemple 2.

Ceci est résumé par la notation « *MgO puis NaOH* » qui indique que l'étape de culture aérobie a lieu avec ajout de MgO, suivie d'une étape de fermentation anaérobie avec ajout de NaOH.

Le tableau ci-dessous compare les résultats obtenus dans ces conditions de régulation de pH (« MgO puis NaOH») à ceux obtenus lorsque seul du MgO, ou seul du NaOH, est utilisé dans les deux phases (« MgO puis MgO» ou «NaOH puis NaOH »).

| | **Rendement Biomasse** | **Productivité volumique Acide Succinique** | **Rendement Acide Succinique** | **Productivité spécifique Acide Succinique** | **Co-produits** |
|---|---|---|---|---|---|
| | Y x/g | PV sa | Y sa/g | PS sa | Acides malique + pyruvique |
| | % glucose aérobie | g/L/h anaérobie | % glucose anaérobie | g/g/h anaérobie | % succinique |
| **« MgO puis NaOH »** | 35 | 1,8 | 95 | 0.37 | 20 |
| **«MgO puis MgO»** | 35 | 2,3 | 103 | 0.48 | 21 |
| **« NaOH puis NaOH »** | 25 | 0.9 | 91 | 0,24 | 26 |

| | | | | | |
|---|---|---|---|---|---|
| sa = acide succinique | | | | | |

Ces résultats montrent qu'il est possible d'obtenir un effet positif sur les performances en phase de production en régulant le pH à l'aide de MgO uniquement au cours de la phase de croissance.

### Exemple 4 : Obtention d'acide succinique par fermentation anaérobie et acidification

La souche utilisée est la souche SBS550MG - pHL413. Le procédé de production d'acide succinique de l'Exemple 2 (avec MgO comme agent de régulation du pH) est suivi d'une étape d'acidification par ajout de différents acides :
- Acide ortho phosphorique : Purification par formation et précipitation de magnésium phosphate tribasique (très insoluble). On ajoute 1 mole d' H₃PO₄ pour 1 mole de succinique en phase aqueuse. Ceci conduit à la formation de magnésium phosphate monobasique très soluble.
- Acide oxalique : Purification par formation et précipitation d'oxalate de magnésium très insoluble. Il n'y a pas de perte d'acide succinique. On obtient rapidement l'acide succinique débarrasse de son contre-ion.

### Exemple 5 : Obtention décide succinique par fermentation anaérobies et conversion en succinate de sodium

La souche utilisée est la souche SBS550MG - pHL413. Le procédé de production d'acide succinique de l'Exemple 2 (avec MgO comme agent de régulation du pH) est suivi d'une étape de formation de carbonate de magnésium et de succinate de sodium, par ajout de carbonate de sodium.

Le carbonate de magnésium, insoluble, précipite, Il n'y a pas de pertes d'acide succinique. Le carbonate de magnésium peut être traité dans un four à haute température, par exemple un four à > 700°C. Il en résulte du MgO et du CO₂, dont l'un au moins peut être recyclé. Le carbonate de magnésium peut ensuite avantageusement être valorisé.

Le succinate de sodium peut être traité par électrolyse donnant de la soude et de l'acide succinique, qui peut être cristallisé. Cette soude peut être reconvertie, avec le CO₂ précédemment émis du four haute température, pour former du carbonate de sodium.

L'ensemble des étapes est représente à la Figure 3.

### Exemple 6 : Obtention d'acide succinique par fermentation anaérobie, purification éthanolique et cristallisation

Le procédé de production d'acide succinique de l'Exemple 1 (avec NaOH comme agent de régulation du pH) est suivi d'une étape de purification éthanolique comme décrit ci-dessous:
- centrifugation du milieu de fermentation (moût) (5000g, 15', 20°C) (élimination de la biomasse),
- ajout d'acide sulfurique 95% au surnageant jusqu'à obtention d'un pH 1,3,
- filtration du moût sur Büchner avec plaque SEITZ EK et terre de filtration FW20 (élimination d'un précipite protéique),
- concentration du filtrat par évaporation sous vide (facteur de concentration oscille entre environ 2 et 8),
- ajout d'éthanol 95%, 2 volumes d'éthanol pour 1 volume du filtrat concentré pour provoquer la précipitation des sels (l'acide succinique reste soluble),
- séparation du précipité salin par filtration sur une membrane millipore 3 microns,
- récupération de l'ethanol par évaporation sous vide,
- traitement charbon actif (2%/sec de PUREFLOW C - 80°C - 1 heure) puis filtration sur plaque SEITZ EK et terre de filtration FW20,
- évapocristallisation (Tp bain marie 75°c - Pression résiduelle 60 mbars - MS masse cuite environ 50%),
- refroidissement de la masse cuite sous agitation à 20°C,
- séparation des cristaux par filtration sur une membrane millipore 3 microns,
- clairçage des cristaux avec de l'eau déminéralisée,
- séchage des cristaux une nuit à 60°c sous vide.

## Revendications

1. Procédé de production d'acide succinique, comprenant :
(a) une étape de culture d'une souche d'*Escherichia coli* en conditions aérobies, lors de laquelle le pH est régulé par ajout, dans le milieu de culture, d'un composé de magnésium,
(b) une étape de production d'ions succinate par fermentation de la souche cultivée à l'étape (a) en conditions anaérobies en présence de CO₂,
(c) une étape de conversion des ions succinate formés à l'étape (b) en acide succinique.

2. Procédé selon la revendication 2, dans lequel à l'étape (a), le composé de magnésium est choisi parmi l'oxyde de magnésium, l'hydroxyde de magnésium, et le carbonate de magnésium.

3. Procédé selon la revendication 1 ou 2, dans lequel lors de l'étape (b), le pH est régulé par ajout, dans le milieu de fermentation, d'un composé choisi dans le groupe constitué des composés du magnésium, des composés du calcium, des composés du potassium, des composés d'ammonium et des composés du sodium, et de leurs mélanges.

4. Procédé selon l'une quelconque des revendications 1-3, dans lequel l'étape (b) se déroule à un pH dans un intervalle de 6,0-7,0, de préférence 6,4-6,8.

5. Procédé selon l'une quelconque des revendications 1-4, dans lequel l'étape (c) comprend une acidification.

6. Procédé selon la revendication 5, dans lequel l'acidification est réalisée par ajout d'au moins un acide choisi parmi l'acide ortho-phosphorique, l'acide oxalique, l'acide sulfurique.

7. Procédé selon l'une quelconque des revendications 1-6, dans lequel, lors de l'étape (b), le pH est régulé par ajout, dans le milieu de fermentation, d'un composé choisi dans le groupe constitué des composés du magnésium, formant ainsi du succinate de magnésium, et l'étape (c) comprend :
(c-1) une étape de conversion de succinate de magnésium formé à l'étape (b) en succinate de sodium, et
(c-2) une étape de conversion, par électrodialyse bipolaire, du succinate de sodium formé à l'étape (c-1) en acide succinique.

8. Procédé selon l'une quelconque des revendications 1-7, dans lequel la souche *d'Escherichia coli* possède le génotype Δ*adhE* Δ*ldhA* Δ*iclR* Δ*ackpta* PYC.

9. Procédé selon la revendication 8, dans lequel la souche *d'Escherichia coli* est la souche SBS550MG - pHL413.

10. Procédé d'obtention d'acide succinique, comprenant :
- un procédé de production d'acide succinique selon l'une quelconque des revendications 1-9 ;
- une étape de purification de l'acide succinique ; et
- optionnellement, une étape de cristallisation de l'acide succinique.

11. Procédé d'obtention d'acide succinique selon la revendication 10, **caractérisé en ce que** l'étape de purification de l'acide succinique est une purification éthanolique.
